# EUROPEAN PATENT APPLICATION

(11) **EP 2 927 220 A1**
(43) Date of publication of application: **07.10.2015**
(21) Application number: 13857920.6
(22) Date of filing: 09.09.2013
(51) Int. Cl.: C07D 319/08, C07C 45/65, C07C 49/747

(54) **METHOD FOR PRODUCING CYCLOPENTANONE DERIVATIVE, INTERMEDIATE COMPOUND, AND METHOD FOR PRODUCING INTERMEDIATE COMPOUND**

(30) Priority: 28.11.2012 JP 2012260333; 06.12.2012 JP 2012267707
(71) Applicant: Kureha Corporation, Chuo-ku Tokyo 103-8552 (JP)
(72) Inventor: KIKUMOTO, Shigeyuki, Tokyo 103-8552 (JP); SHIMOKAWARA, Takashi, Tokyo 103-8552 (JP)
(74) Representative: BRP Renaud & Partner mbB Rechtsanwälte Patentanwälte Steuerberater
(86) International application number: PCT/JP2013/074281
(87) International publication number: WO 2014/083911

(57) **Abstract**

A method for producing a cyclopentanone derivative represented by general formula (III) below, wherein a cyclopentanone derivative represented by general formula (III) below can be produced with higher yield since the method comprises a step in which a compound represented by general formula (II) below is obtained by reacting a compound represented by general formula (I) below with an acid.

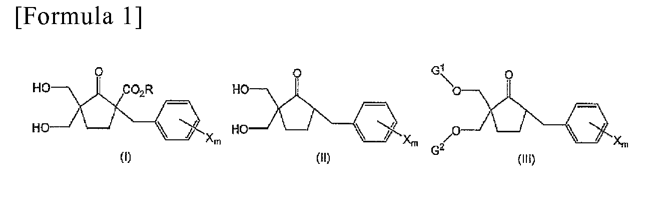

## Description

### TECHNICAL FIELD

The present invention relates to a novel production method for a cyclopentanone derivative, an intermediate compound of the cyclopentanone derivative, and a production method of the intermediate compound.

### BACKGROUND ART

A certain type of 2-(halogenated hydrocarbon substituted)-5-benzyl-1-azolylmethylcyclopentanol derivative is described in Patent Document 1 as a compound that can be used as an active ingredient for agricultural and horticultural chemicals, industrial material protectants, and the like. A method for producing a 2-benzyl-5,5-bis(hydroxymethyl)-cyclopentanone derivative having a protected hydroxy group from a 1-benzyl-2-oxocyclopentane carboxylic acid alkyl ester derivative is also described in this document as a part of a step in the production method for this derivative.

### CITATION LIST

### Patent Literature

[Patent Document 1] WO/2011/070771 (published June 16, 2011)

### SUMMARY OF INVENTION

### Technical Problem

In order to mass-produce a 2-benzyl-5,5-bis(hydroxymethyl)-cyclopentanone derivative having a protected hydroxy group less expensively, it is necessary to improve the yield when producing a 2-benzyl-5,5-bis(hydroxymethyl)-cyclopentanone derivative having a protected hydroxy group from a 1-benzyl-2-oxocyclopentane carboxylic acid alkyl ester derivative.

The invention of the present application was conceived in light of the problem described above, and an object of the present invention is to provide a method for more efficiently producing a 2-benzyl-5,5-bis(hydroxymethyl)-cyclopentanone derivative having a protected hydroxy group, which is an intermediate compound of a compound that can be used as an active ingredient for agricultural and horticultural chemicals, industrial material protectants, and the like.

### Solution to Problem

An embodiment of the present invention is a production method for a cyclopentanone derivative represented by general formula (III) below: 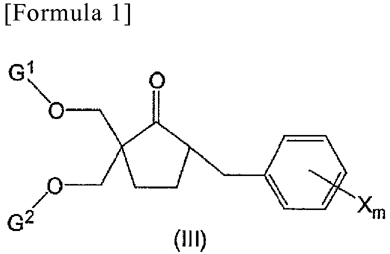 in formula (III), X is a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group; m is an integer from 0 to 5; a plurality of X moieties may be the same or different when m is 2 or greater; G¹ and G² each represents a protecting group that dissociates under acidic conditions, G¹ and G² may be the same or different, and G¹ and G² may bond with one another to form a ring;
the method comprising a step of obtaining a compound represented by general formula (II) below by reacting a compound represented by general formula (I) below with an acid: 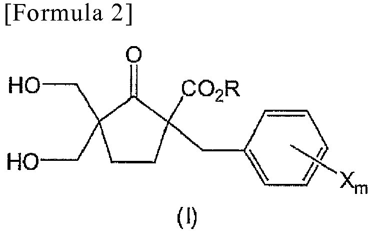 in formula (I), X and m are each the same as X and m in formula (III), and R is an alkyl group having from 1 to 4 carbons; and 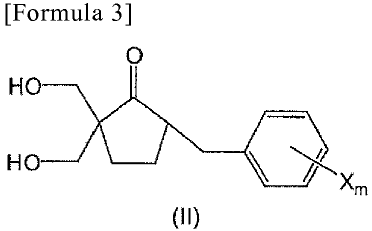 in formula (II), X and m are each the same as X and m in formula (III).
Another embodiment of the present invention is a production method for a compound represented by general formula (II) above, wherein the compound represented by general formula (I) above is reacted with an acid.

Yet another embodiment of the present invention is a production method for a cyclopentanone derivative represented by general formula (III) above, wherein the hydroxy groups of the compound represented by general formula (II) above are protected by protecting groups that dissociate under acidic conditions.

The present invention also includes the compound represented by general formula (II) above.

### ADVANTAGEOUS EFFECTS OF INVENTION

With the present invention, it is possible to produce a 2-benzyl-5,5-bis(hydroxymethyl)-cyclopentanone derivative having a protected hydroxy group with higher yield.

### DESCRIPTION OF EMBODIMENTS

An embodiment of the production method for a cyclopentanone derivative according to the present invention will be described hereinafter.

The production method for a cyclopentanone derivative according to an embodiment of the present invention is a production method for a cyclopentanone derivative represented by general formula (III) below (hereafter called "compound (III)"). 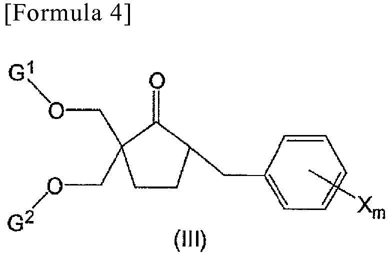 In formula (III), X is a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group; m is an integer from 0 to 5; a plurality of X moieties may be the same or different when m is 2 or greater; G¹ and G² each represents a protecting group that dissociates under acidic conditions, G¹ and G² may be the same or different, and G¹ and G² may bond with one another to form a ring;

### [1. Compound (III)]

Compound (III) will be described below.

Compound (III) is an intermediate compound of a compound that can be suitably used as an active ingredient for agricultural and horticultural chemicals and industrial material protectants.

In formula (III), X is a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group.

Specific examples of halogen atoms in X include fluorine atoms, chlorine atoms, bromine atoms, and iodine atoms. Of these, fluorine atoms, chlorine atoms, and bromine atoms are preferable, and chlorine atoms are more preferable.

Specific examples of the alkyl group having from 1 to 4 carbons of X include a methyl group, ethyl group, n-propyl group, isopropyl group, n-butyl group, isobutyl group, sec-butyl group, and tert-butyl group. Of these, alkyl groups having from 1 to 3 carbons are preferable, and alkyl groups having 1 or 2 carbons are more preferable. Methyl groups are even more preferable.

The haloalkyl group having from 1 to 4 carbons in X is an alkyl group substituted with 1 or 2 or more of the same or different halogen atoms, examples of which include dichloromethyl groups, trichloromethyl groups, 2-chloroethyl groups, 1-chloroethyl groups, 2,2-dichloroethyl groups, 1,2-dichloroethyl groups, 2,2,2-trichloroethyl groups, 3-chloropropyl groups, 2,3-dichloropropyl groups, 1-chloro-1-methylethyl groups, 2-chloro-1-methylethyl groups, 2-chloropropyl groups, 4-chlorobutyl groups, fluoromethyl groups, difluoromethyl groups, trifluoromethyl groups, 2-fluoroethyl groups, 1-fluoroethyl groups, 2,2-difluoroethyl groups, 1,2-difluoroethyl groups, 2,2,2-trifluoroethyl groups, 3-fluoropropyl groups, 2,3-difluoropropyl groups, 1-fluoro-1-methylethyl groups, 2-fluoro-1-methylethyl groups, 2-fluoropropyl groups, 3,3,3-trifluoropropyl groups, 2,2,3,3-tetrafluoropropyl groups, 2,2,3,3,3-pentafluoropropyl groups, 4-fluorobutyl groups, dibromomethyl groups, tribromomethyl groups, 2-bromoethyl groups, 2,2-dibromoethyl groups, 1,2-dibromoethyl groups, 2,2,2-tribromoethyl groups, 3-bromopropyl groups, 2,3-dibromopropyl groups, 1-bromo-1-methylethyl groups, 2-bromo-1-methylethyl groups, 2-bromopropyl groups, diiodomethyl groups, 2,2-diiodoethyl groups, 1,2-diiodoethyl groups, 2,2,2-triiodoethyl groups, 2,3-diiodopropyl groups, 1-iodo-1-methylethyl groups, 2-iodo-1-methylethyl groups, and the like. Of these, haloalkyl groups having from 1 to 3 carbons are preferable, and haloalkyl groups having 1 or 2 carbons are more preferable. Trihaloalkyl groups having 1 carbon are even more preferable.

Examples of alkoxy groups having from 1 to 4 carbons in X include methoxy groups, ethoxy groups, n-propoxy groups, and the like. Of these, alkoxy groups having from 1 to 3 carbons are preferable, and alkoxy groups having 1 or 2 carbons are more preferable. Methoxy groups are even more preferable.

The haloalkoxy group having from 1 to 4 carbons in X is an alkoxy group substituted with 1 or 2 or more of the same or different halogen atoms, examples of which include trifluoromethoxy groups, difluoromethoxy groups, 1,1,2,2,2-pentafluoroethoxy groups, 2,2,2-tifluoroethoxy groups, and the like. Of these, haloalkoxy groups having from 1 to 3 carbons are preferable, and haloalkoxy groups having 1 or 2 carbons are more preferable. Dihalomethoxy groups and trihalomethoxy groups having 1 carbon are even more preferable.

X is preferably a halogen atom, an alkyl group having from 1 to 3 carbons or a haloalkyl group having from 1 to 3 carbons, more preferably a halogen atom, a methyl group, a trifluoromethyl group, a trifluoromethoxy group, or a difluoromethoxy group, even more preferably a halogen atom, and particularly preferably a chlorine atom.

In formula (III), m is an integer from 0 to 5. m is preferably an integer from 0 to 3, more preferably an integer from 0 to 2, and even more preferably 0 or 1. When m is an integer of 2 or greater, a plurality of X moieties may be the same or different from one another. When m is an integer of 1 or greater, X may be positioned at any of the positions 2 to 6 of a benzene ring. When m is 1, a position forming 4-substituted benzyl is preferable.

In formula (III), G¹ and G² are each a protecting group that dissociates under acidic conditions. G¹ and G² are protecting groups that protect hydroxy groups. G¹ and G² may be the same or different from one another. In addition, G¹ and G² may bond with one another to form a ring. G¹ and G² are not particularly limited as long as they dissociate under acidic conditions.

When G¹ and G² do not bond with one another, compound (III) can be expressed by general formula (IIIb) below.

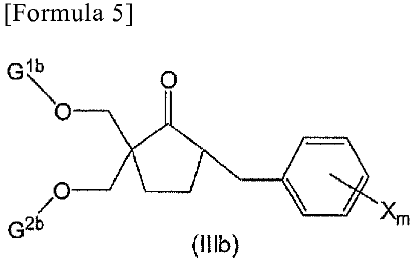

In formula (IIIb), X and m are each the same as X and m in formula (III).

In formula (IIIb), G^{1b} and G^{2b} are each independently an alkoxymethyl group having from 1 to 4 carbons in the alkoxy moiety, an alkoxyethyl group having from 1 to 4 carbons in the alkoxy moiety, an alkyl group having from 1 to 4 carbons, an allyl group, a substituted or unsubstituted benzyl group, a substituted or unsubstituted tetrahydropyranyl group, or a substituted or unsubstituted tetrahydrofuranyl group.

Examples of alkoxymethyl groups having from 1 to 4 carbons in the alkoxy moiety in G^{1b} and G^{2b} include methoxymethyl groups, ethoxymethyl groups, and the like.

Examples of alkoxyethyl groups having from 1 to 4 carbons in the alkoxy moiety in G^{1b} and G^{2b} include 1-ethoxyethyl groups, 1-methyl-1-methoxyethyl groups, and the like.

Specific examples of alkyl groups having from 1 to 4 carbons in G^{1b} and G^{2b} include methyl groups, ethyl groups, n-propyl groups, isopropyl groups, n-butyl groups, isobutyl groups, sec-butyl groups, and tert-butyl groups.

Meanwhile, examples of protecting groups when G¹ and G² bond with one another to form a ring include, but are not limited to, methylene acetal, ethylidene acetal, t-butylmethylidene ketal, 1-t-butylethylidene ketal, 1-phenylethylidene ketal, acrolein acetal, isopropylidene ketal (acetonide), cyclopentylidene ketal, cyclohexylidene ketal, cycloheptylidene ketal, benzylidene acetal, p-methoxybenzylidene acetal, 2,4-dimethoxybenzylidene ketal, 3,4-dimethoxybenzylidene ketal, 2-nitrobenzylidene acetal, 4-nitrobenzylidene acetal, mesitylene acetal, 1-naphthaldehyde acetal, benzophenone ketal, camphor ketal, menthone, methoxymethylene acetal, ethoxymethylene acetal, dimethoxymethylene orthoester, 1-methoxyethylidene orthoester, 1-ethoxyethylidene orthoester, methylidene orthoester, phthalide orthoester, 1,2-dimethoxyethylidene orthoester, α-methoxybenzylidene orthoester, 2-oxacyclopentylidene orthoester, butane-2,3-bis-acetal, cyclohexane-1,2-diacetal, bis-dihydropyran ketal, di-t-butylsilylene, 1,3-(1,1,3,3-tetraisopropyl)disiloxanilidene, 1,1,3,3-tetra-t-butoxydisiloxanilidene, and the like.

Of these, when G¹ and G² bond with one another to form a ring, compound (III) is preferably a compound represented by general formula (IIIa) below.

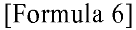

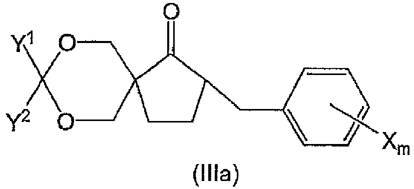

In formula (IIIa), X and m are each the same as X and m in formula (III).

In formula (IIIa), Y¹ and Y² are each independently a hydrogen atom, an alkyl group having from 1 to 4 carbons, an alkenyl group having from 1 to 4 carbons, a phenyl group, a naphthyl group, or a benzyl group. The phenyl parts of the phenyl groups, naphthyl groups, and benzyl groups of Y¹ and Y² may be further substituted with alkyl groups having from 1 to 4 carbons such as methyl groups and ethyl groups; alkoxy groups having from 1 to 4 carbons such as methoxy groups and ethoxy groups; nitro groups; or halogen atoms such as fluorine atoms and chlorine atoms. In addition, Y¹ and Y² may bond with one another to form a ring. Of these, Y¹ and Y² are more preferably independently hydrogen atoms or alkyl groups having from 1 to 4 carbons such as methyl groups, ethyl groups, and n-propyl groups, and even more preferably independently hydrogen atoms or methyl groups. It is particularly preferable for both Y¹ and Y² to be methyl groups or to be hydrogen atoms.

### [2. Compound (III) Production Method]

The production method for compound (III) of the present invention is a method comprising a step of obtaining a compound represented by general formula (II) below (hereafter called "compound (II)") by reacting a compound represented by general formula (I) below (hereafter called "compound (I)") with an acid (step 1). 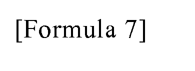 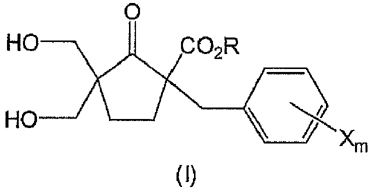 In formula (I), X and m are each the same as X and m in formula (III), and R is an alkyl group having from 1 to 4 carbons; and

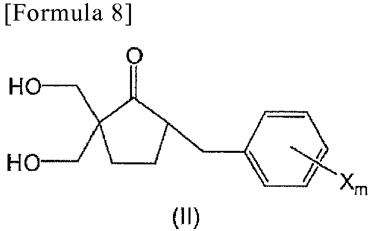

in formula (II), X and m are each the same as X and m in formula (III).

The other steps of the production method for compound (III) of the present invention are not particularly limited as long as the method includes step 1, but an example of a preferable production method is a method which further includes a step of obtaining compound (III) by protecting the hydroxy groups of compound (II) with protecting groups (step 2) in addition to step 1, as described in reaction scheme 1 below. An embodiment of the production method for compound (III) will be described below using the reaction described in reaction scheme 1 as an example.

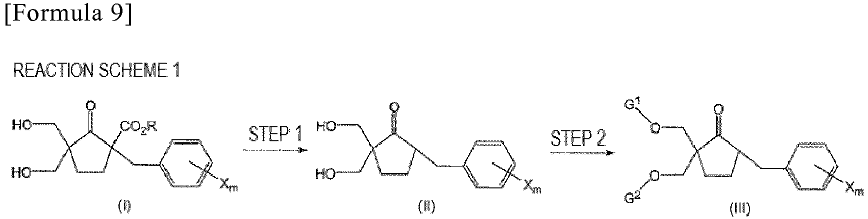

### (Step 1: Hydrolysis/decarboxylation step)

Step 1 is a step of obtaining compound (II) by reacting compound (I) with an acid. In step 1, hydrolysis and decarboxylation are performed.

In formula (I), X and m are each the same as X and m in formula (III).

In formula (I), R is an alkyl group having from 1 to 4 carbons. Specific examples of the alkyl group having from 1 to 4 carbons of R include a methyl group, ethyl group, n-propyl group, isopropyl group, n-butyl group, isobutyl group, sec-butyl group, and tert-butyl group. Of these, alkyl groups having from 1 to 3 carbons are preferable, and alkyl groups having 1 or 2 carbons are more preferable. Methyl groups are even more preferable.

A compound produced by a publicly known method (for example, the method described in Patent Document 1) may be used as compound (I).

Examples of acids in step 1 include inorganic acids such as hydrochloric acid, hydrobromic acid, hydroiodic acid, hydrofluoric acid, chloric acid, perchloric acid, sulfuric acid, nitric acid, phosphoric acid, hexafluorophosphoric acid, and tetrafluoroboric acid; and organic acids such as acetic acid, trifluoroacetic acid, formic acid, benzoic acid, methanesulfonic acid, ethanesulfonic acid, benzenesulfonic acid, p-toluenesulfonic acid, p-chlorobenzenesulfonic acid, trifluoromethanesulfonic acid, and camphorsulfonic acid.

The amount of acid used is, for example, from 0 times to 20 times (excluding 0 times) the molar quantity of compound (I) and is preferably from 0.001 times to 10 times the molar quantity of compound (I).

The reaction temperature is, for example, from -20°C to 200°C and is preferably from 0°C to 150°C. The reaction time is, for example, from 0.1 hours to several days and is preferably from 0.5 hours to 2 days.

The solvent used in step 1 is not particularly limited, and examples of solvents include water, toluene, and the like.

Compound (II) obtained in step 1 is a novel compound. Therefore, the present invention also provides compound (II) and a production method for compound (II) by means of step 1.

### (Step 2: Protection step)

Step 2 is a step of obtaining compound (III) by protecting the hydroxy groups of compound (II) with protecting groups. Here, the protecting groups that are introduced are protecting groups that dissociate under acidic conditions.

The introduction of G¹ and G² is realized by reacting a compound for introducing protecting groups with compound (II) in the presence of an acid.

The compound for introducing protecting groups is not particularly limited as long as the compound is capable of introducing the protecting groups described above, but examples include acetone dimethyl acetal, isobutene, acetone, dialkoxymethane; and the like.

Examples of acids in step 2 include inorganic acids such as hydrochloric acid, hydrobromic acid, hydroiodic acid, hydrofluoric acid, chloric acid, perchloric acid, sulfuric acid, nitric acid, phosphoric acid, hexafluorophosphoric acid, and tetrafluoroboric acid; organic acids such as acetic acid, trifluoroacetic acid, formic acid, benzoic acid, methanesulfonic acid, ethanesulfonic acid, benzenesulfonic acid, p-toluenesulfonic acid, p-chlorobenzenesulfonic acid, trifluoromethanesulfonic acid, and camphorsulfonic acid; and the like. Some or all of the acids used for step 1 may be used as the acids for step 2. Furthermore, step 1 and step 2 are preferably performed using the same type of acid. In this case, preferable acids used in both step 1 and step 2 include sulfuric acid and sulfonic acid, and the acid is more preferably p-toluene sulfonic acid.

The amount of acid used is, for example, from 0 times to 10 times (excluding 0 times) the molar quantity of compound (I) and is preferably from 0.001 times to 5 times the molar quantity of compound (II).

The amount of the compound used to introduce protecting groups can be set appropriately in accordance with the types of the compound, the acid used, and the compound (III), but the amount is, for example, from 0.5 times to 50 times the molar quantity of compound (II) and is preferably from 0.8 times to 10 times the molar quantity of compound (II).

The solvent used in step 2 is not particularly limited, but examples include acetone, toluene, tetrahydrofuran, and the like.

When (a) an alkoxymethyl group is introduced, (b) a t-butyl group is introduced, or (c) two hydroxy groups are simultaneously protected with acetal or ketal as a protecting group, the respective methods described below are preferably used.

First, the case in which (a) an alkoxymethyl group is introduced will be described.

When an alkoxymethyl group is introduced, it is preferable to use a method of subjecting the hydroxy groups in compound (II) to acetal exchange using formaldehyde dialkyl acetal.

Examples of acids that can be used in this case include inorganic acids such as hydrochloric acid, phosphoric acid (including compounds in which acidic moieties are produced by the addition of alcohol or water, such as diphosphorus pentaoxide), and sulfuric acid, and organic acids such as p-toluenesulfonic acid. Formaldehyde dialkyl acetal is preferably used in the presence of an acid in a solvent or without a solvent. It is more preferable to add a compound capable of removing alcohol that is produced (for example, diphosphorus pentaoxide).

The amount of formaldehyde dialkyl acetal used is, for example, from 0.5 times to 50 times the molar quantity of compound (II) and is preferably from 0.8 times to 10 times the molar quantity of compound (II). The amount of acid used is, for example, from 0.01 times to 10 times the molar quantity of compound (II) and is preferably from 0.05 times to 5 times the molar quantity of compound (II).

The reaction temperature is, for example, from 0°C to 250°C and is preferably from 0° to 150°C. The reaction time is, for example, from 0.1 hours to several days and is preferably from 0.5 hours to 2 days.

Next, the case in which (b) t-butyl groups are introduced will be described.

When t-butyl groups are introduced, it is preferable to use a method of introducing t-butyl groups into the hydroxy groups in compound (II) using isobutene.

Examples of acids that can be used in this case include inorganic acids such as hydrochloric acid, phosphoric acid, and sulfuric acid, and organic acids such as p-toluenesulfonic acid and trifluoroacetic acid. It is preferable to react compound (II) and isobutene in a solvent.

The amount of isobutene used is, for example, from 0.5 times to 100 times the molar quantity of compound (II) and is preferably from 0.8 times to 20 times the molar quantity of compound (II). The amount of acid used is, for example, from 0.01 times to 10 times the molar quantity of compound (II) and is preferably from 0.05 times to 5 times the molar quantity of compound (II).

The reaction temperature is, for example, from 0°C to 200°C and is preferably from 0° to 100°C. The reaction time is, for example, from 0.1 hours to several days and is preferably from 0.5 hours to 2 days.

Finally, the case in which (c) two hydroxy groups are simultaneously protected with acetal or ketal will be described.

When two hydroxy groups are simultaneously protected with acetal or ketal, it is preferable to use a method of reacting an appropriate aldehyde or ketone with compound (II) in the presence of an acid. This makes it possible to introduce protecting groups in which G¹ and G² bond with one another to form a ring.

When two hydroxy groups are simultaneously protected with acetal or ketal and, for example, the protecting group that is introduced is isopropylidene ketal, it is preferable to react compound (II) with acetone or acetone dimethyl acetal in the presence of an acid in a solvent. Examples of acids that can be used in this case include inorganic acids such as hydrochloric acid, phosphoric acid, and sulfuric acid, and organic acids such as p-toluenesulfonic acid and trifluoroacetic acid.

The amount of acetone dimethyl acetal used is, for example, from 0.5 times to 50 times the molar quantity of compound (II) and is preferably from 0.8 times to 10 times the molar quantity of compound (II). The amount of acid used is, for example, from 0 times to 100 times (excluding 0 times) the molar quantity of compound (III) and is preferably from 0.001 times to 50 times the molar quantity of compound (III).

Compound (III) obtained above is suitably used in the synthesis of an azole derivative, which is an active ingredient of agricultural and horticultural chemicals and industrial material protectants described in Patent Document 1. The specific production of an azole derivative from compound (III) can be realized in accordance with the method described in Patent Document 1.

Compound (II) used in step 2 is a novel compound. Therefore, the present invention also provides a production method for compound (III) by means of step 2.

### (Advantages of production method of the present invention)

The advantages of the production method for compound (III) including step 1 will be described. Using the method described in Patent Document 1 as a reference, an example of a method for producing compound (III) from compound (I) is reaction scheme 2 below. Here, X, m, R, G¹, and G² in compounds described in reaction scheme 2 are the same as X, m, R, G¹, and G² described above.

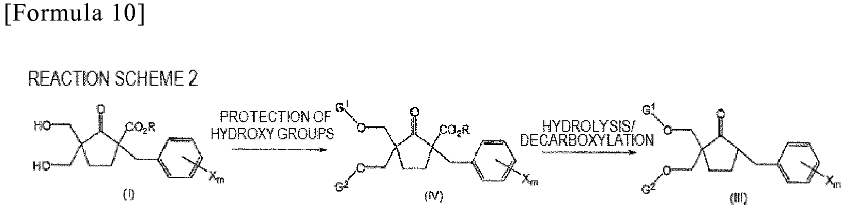

In reaction scheme 2, G¹ and G² in the compound represented by general formula (IV) (hereafter called "compound (IV)") are protecting groups that dissociate under acidic conditions. Therefore, the hydrolysis and decarboxylation reactions in the step of producing compound (III) from compound (IV) is performed under basic conditions. However, as a result of conducting various investigations, it was found that when compound (IV) is hydrolyzed and decarboxylated under basic conditions, the yield of compound (III) decreases due to the occurrence of side reactions involving the opening of cyclopentane rings.

In contrast, in the production method for compound (III) including step 1 of the present invention, hydrolysis and decarboxylation are performed under acidic conditions by undergoing step 1 before introducing protecting groups. As a result, in the hydrolysis/decarboxylation, it is possible to avoid reactions by which side reactions involving the opening of cyclopentane rings may be caused. Accordingly, the production method for compound (III) of the present invention is excellent from the perspective of the yield of compound (III) since by-products are unlikely to be generated.

Furthermore, in the production method for compound (III) that further includes step 2, the number of steps to produce compound (III) from compound (I) is the same as that of reaction scheme 2. Therefore, decrease in yield as a whole due to increase in the number of steps can be avoided.

Furthermore, in reaction scheme 2, the step of producing compound (IV) from compound (I) is performed under acidic conditions, while the step of producing compound (III) from compound (IV) is performed under basic conditions. Therefore, before the step of producing compound (III) from compound (IV) under basic conditions, an operation such as neutralization and water washing is required, and in the case where acid remains, yield of compound (III) can be decreased.

On the other hand, step 1 and step 2 of the present invention are both performed under acidic conditions. Therefore, even when acid used in step 1 remains in step 2, yield of compound (III) is less likely to be reduced. For cases where the same type of acid is used in both step 1 and step 2, step 1 and step 2 can be performed in one pot. Therefore, the production method of compound (III) of the present invention can reduce waste. Furthermore, the production method can simplify the production of compound (III) since an operation such as neutralization and water washing is not needed in between step 1 and step 2.

### [3. Summary]

An embodiment of the present invention is a production method for a cyclopentanone derivative represented by general formula (III) below: 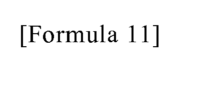 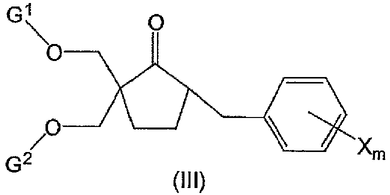 in formula (III), X is a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group; m is an integer from 0 to 5; a plurality of X moieties may be the same or different when m is 2 or greater; G¹ and G² each represents a protecting group that dissociates under acidic conditions, G¹ and G² may be the same or different, and G¹ and G² may bond with one another to form a ring);
the method comprising a step of obtaining a compound represented by general formula (II) below by reacting a compound represented by general formula (I) below with an acid: 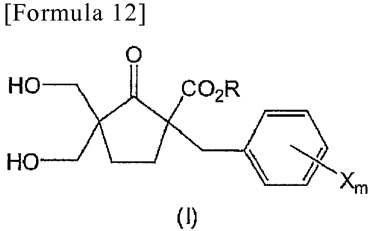 in formula (I), X and m are each the same as X and m in formula (III), and R is an alkyl group having from 1 to 4 carbons; and 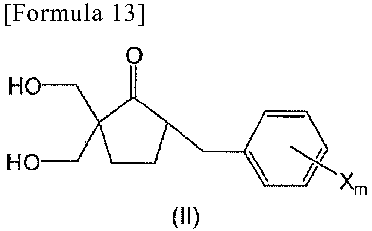 in formula (II), X and m are each the same as X and m in formula (III).
The production method of the present invention preferably further comprises a step of obtaining a cyclopentanone derivative represented by general formula (III) above by protecting the hydroxy groups of a compound represented by general formula (II) above with protecting groups that dissociate under acidic conditions:

In the production method of the present invention, there are some cases where the cyclopentanone derivative represented by general formula (III) above is preferably a compound represented by general formula (IIIa) below:

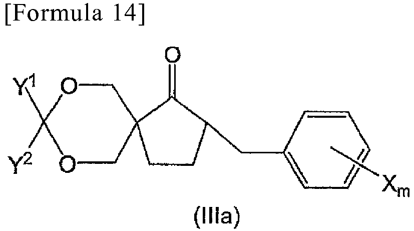

in formula (IIIa), X and m are each the same as X and m in formula (III); Y¹ and Y² are each independently a hydrogen atom, an alkyl group having from 1 to 4 carbons, an alkenyl group having from 1 to 4 carbons, or a substituted or unsubstituted phenyl group, naphthyl group, or benzyl group; and Y¹ and Y² may bond with one another to form a ring.
In general formula (IIIa) above, Y¹ and Y² are preferably each independently a hydrogen atom or an alkyl group having from 1 to 4 carbons.

In general formula (IIIa) above, both Y¹ and Y² are more preferably methyl groups or hydrogen atoms.

In general formula (III) above of the production method of the present invention, m is preferably an integer from 0 to 3, and when m is 1 or greater, X is preferably a halogen atom, an alkyl group having from 1 to 3 carbons, or a haloalkyl group having from 1 to 3 carbons.

In general formula (III) of the production method of the present invention, m is more preferably an integer from 0 to 2, and when m is 1 or 2, X is more preferably a halogen atom.

Another embodiment of the present invention is a production method for a compound represented by general formula (II) above, wherein the compound represented by general formula (I) above is reacted with an acid.

Yet another embodiment of the present invention is a production method for a cyclopentanone derivative represented by general formula (III) above, wherein the hydroxy groups of a compound represented by general formula (II) above are protected by protecting groups that dissociate under acidic conditions.

The present invention also include a compound represented by general formula (II) above.

Embodiments of the present invention will be described in further detail hereinafter using examples. Of course, the present invention is not limited to the examples below, and it goes without saying that various modes are possible with regard to the details thereof. Furthermore, the present invention is not limited to the embodiments described above, and various modifications are possible within the scope indicated in the claims. Embodiments obtained by appropriately combining the technical means disclosed by the embodiments are also included in the technical scope of the present invention. In addition, all of the documents cited in this specification are hereby incorporated by reference.

### EXAMPLES

### (Working Example 1)

In this working example, methyl 1-(4-chlorobenzyl)-3,3-bishydroxymethyl-2-oxocyclopentane carboxylate (compound (2)) was synthesized from methyl 1-(4-(chlorobenzyl)-2-oxocyclopentane carboxylate (compound (1)) in accordance with reaction scheme 3 described below. Thereafter, 2-(4-chlorobenzyl)-8,8-dimethyl-7,9-dioxaspiro[4,5]decan-1-one (compound (4)) was synthesized from compound (2). Furthermore, 5-(4-chlorobenzyl)-2,2-bis(hydroxymethyl)-1-(1H-1,2,4-triazol-1-ylmethyl)cyclopentanol (compound (6)) was synthesized from compound (4).

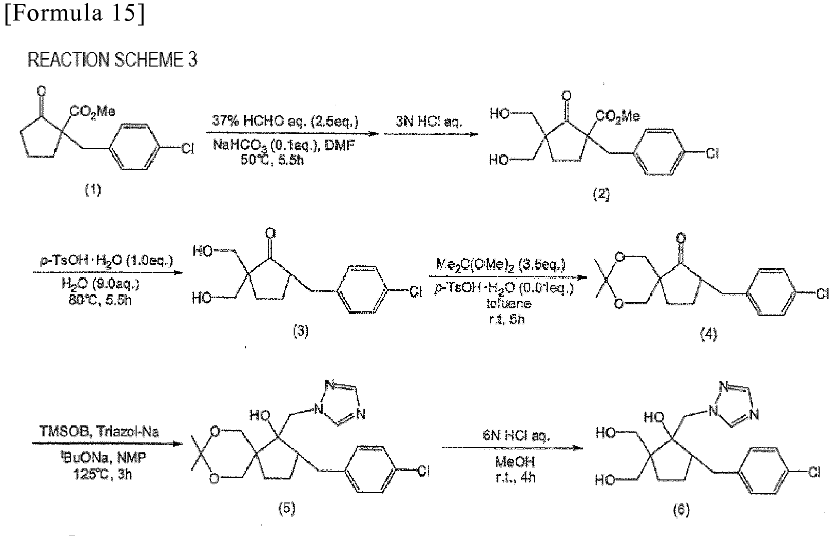

### <Reference Production Example 1-1: Synthesis of methyl 1-(4-chlorobenzyl)-3,3-bishydroxymethyl-2-oxocyclopentane carboxylate (compound (2))>

Methyl 1-(4-(chlorobenzyl)-2-oxocyclopentane carboxylate (compound (1)) (19.1 g) was dissolved in dimethylformamide (10 mL). Then sodium hydrogen carbonate (602 mg) and a 37% formaldehyde aqueous solution (13.5 mL) were added and stirred at 50°C for 5.5 hours. Thereafter, a 3N hydrochloric acid aqueous solution (30 mL) was added to the mixture and stirred at room temperature for a while. Following the completion of the reaction, the solution was extracted with ethyl acetate, and after the solution was washed with saturated saline, the organic layer was dried with anhydrous sodium sulfate. The solvent was distilled out, and the residue was purified by silica gel column chromatography to obtain 22.7 g of the target product (compound (2)) as a colorless crystal.
Yield: 22.7 g
Yield: 97.2%

### <Production Example 1-1: Synthesis of 5-(4-chlorobenzyl)-2,2-bishydroxymethyl-cyclopentanone (compound (3))>

To compound (2) (1.00 g, 3.06 × 10⁻³ mol), p-toluenesulfonic acid monohydrate (582 mg, 3.06 × 10⁻³ mol) and water (496 µL, 2.75 × 10⁻² mol) were added and stirred at 80°C for 5.5 hours. Following the completion of the reaction, the solution was extracted with chloroform, and after the solution was washed with saturated saline, the organic layer was dried with anhydrous sodium sulfate. The solvent was distilled out, and the residue was purified by silica gel column chromatography to obtain 712 mg of the target product (compound (3)) as a colorless crystal.
Yield: 712 mg
Yield: 93.8%
¹H-NMR (400 MHz, CDCl₃):δ = 1.26-1.65(1H, m), 1.78-1.94(2H, m), 2.02-2.09(1H, m), 2.27-2.33(2H, m), 2.50-2.58(1H, m), 2.61(1H, dd, J=13.5, 8.7 Hz), 3.06(1H, dd, J=13.5, 4.1 Hz), 3.54(1H, dd, J=11.1, 7.4 Hz), 3.59(1H, dd, J=11.1, 4.2 Hz), 3.69(1H, dd, J=10.9, 4.1 Hz), 3.77(1H, dd, J=10.9, 7.2 Hz), 7.07-7.10(2H, m), 7.23-7.26(2H, m).

### <Production Example 1-2: Synthesis of 2-(4-chlorobenzyl)-8,8-dimethyl-7,9-dioxaspiro[4.5]decan-1-one (compound (4))>

Compound (3)(497 mg,1.85×10⁻³ mol) was dissolved in toluene (1.5 mL). Then p-toluenesulfonic acid monohydrate (3.50 mg, 1.85 × 10⁻⁵ mol) and acetone dimethyl acetal (567 µL, 4.62 × 10⁻³ mol) were added and stirred at room temperature for 3 hours. Thereafter, acetone dimethyl acetal (226 µL, 1.85 × 10⁻³ mol) was added and stirred at room temperature for 2 hours. Following the completion of the reaction, a saturated sodium hydrogen carbonate aqueous solution was added thereto and stirred for a while. This solution was then extracted with toluene, and after the solution was washed with a saturated sodium hydrogen carbonate aqueous solution and then with saturated saline, the organic layer was dried with anhydrous sodium sulfate. The solvent was distilled out, and the residue was purified by silica gel column chromatography to obtain 524 mg of the target product (compound (4)) as a colorless crystal.
Yield: 524 mg
Yield: 91.7%
¹H-NMR (400 MHz, CDCl₃):δ = 1.38(3H, s), 1.49(3H, s), 1.49-1.59(1H, m), 1.80-1.88(1H, m), 2.04-2.12(1H, m), 2.39-2.50(2H, m), 2.60(1H, dd, J=13.9, 8.4 Hz), 3.00(1H, dd, J=13.9, 4.5 Hz), 3.24(1H, dd, J=11.4, 2.6 Hz), 3.47(1H, dd, J=11.4, 2.6 Hz), 3.78(1H, dd, J=11.4, 1.6 Hz), 4.06(1H, d, J=11.4 Hz), 7.04-7.07(2H, m), 7.22-7.25(2H, m).

### <Reference Production Example 1-2: Synthesis of 2-(4-chlorobenzyl)-8,8-dimethyl-1-(1H-1,2,4-triazol-1-yl methyl)-7,9-dioxaspiro[4,5]decan-1-ol (compound (5))>

First, after NaH (0.91 g (ca. 60% in mineral oil)) was suspended in N-methylpyrrolidone (NMP) (8 mL), 1,2,4-triazole (1.67 g) was added and stirred for 0.5 hours to produce a sodium salt. Next, compound (4) (5.00 g) was added. After this was heated to approximately 90°C (bath temperature), trimethylsulfoxonium bromide (TMSOB) (4.20 g) and t-BuONa (0.77 g) were intermittently added over the course of 1.5 hours and then reacted for 1.5 hours. After the reaction solution was further heated to approximately 125°C (bath temperature) and reacted for 1 hour, saturated ammonium chloride and water were added to the reaction solution and then extracted with ethyl acetate. After the organic layer was dried with anhydrous sodium sulfate, the solvent was distilled out, and the crude product was purified by silica gel column chromatography to obtain the target product (compound (5)) as an isomer mixture consisting of a 93:7 ratio of the cis-form to the trans-form.
Yield: 4.68 g
Yield rate: 74.3%

Reference Production Example 1-3: Synthesis of 5-(4-chlorobenzyl)-2,2-bis(hydroxymethyl)-1-(1H-1,2,4-triazol-1-yl methyl)cyclopentanol (compound (6)) Compound (5) (8.98 g) was dissolved in a mixture of methanol (30 mL) and a 6N hydrochloric acid aqueous solution (40 mL) and stirred for 4 hours at room temperature. After water was added to the mixture, the mixture was neutralized with sodium carbonate and sodium bicarbonate. Next, after this was extracted with ethyl acetate, the organic layer was washed with saturated saline. After the organic layer was dried with anhydrous sodium sulfate, the solvent was distilled out to obtain the target product (compound (6)) as an isomer mixture.
Yield: 7.96 g
Yield rate: 98.7%

### (Working Example 2)

In this working example, 2-(4-chlorobenzyl)-7,9-dioxaspiro[4,5]decan-1-one (compound (7)) was synthesized from compound (3) in accordance with reaction scheme 4 described below.

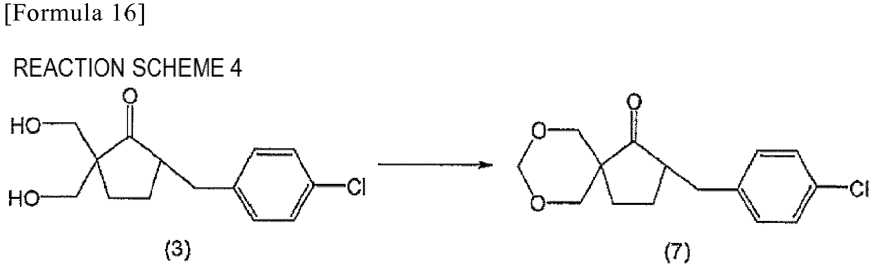

### <Production Example 2-1: Synthesis of 2-(4-chlorobenzyl)-7,9-dioxaspiro[4,5]decan-1-one (compound (7))>

Compound (3) (2.73 g, 1.02 × 10⁻² mol) was dissolved in toluene (8.1 mL), and dimethoxymethane (885 µL, 1.00 × 10⁻² mol) and p-toluenesulfonic acid monohydrate (190 mg, 1.0 × 10⁻³ mol) were added thereto and stirred at 60°C for 5 hours and then at 80°C for 8 hours. Following the completion of the reaction, the solution was washed with a saturated sodium hydrogen carbonate aqueous solution and then with saturated saline, and dried with anhydrous sodium sulfate. The solvent was distilled out, and the residue was purified by silica gel column chromatography to obtain 2.05 g of the target product (compound (7)) as a colorless solid.
Yield: 2.05 g
Yield: 71.7%
¹H-NMR (400 MHz, CDCl₃):δ = 1.51-1.59(1H,m),1.83-1.93(1H,m),2.03-2.14(1H,m),2.37-2.46(1H,m),2.46-2.52(1H,m),2.60(1H,dd,J=13.8,8.3 Hz),3.00(1H,dd,J=13.8,4.4 Hz),3.47(1H,dd,J=11.1,2.3 Hz),3.54(1H,dd,J=11.1,1.5 Hz),3.70(1H,dd,J=11.1,2.3 Hz),3.88(1H,d,J=11.1 Hz),4.67(1H,d,J=6.1 Hz),4.97(1H,d,J=6.1 Hz),7.05(2H,d,J=8.4 Hz),7.23(1H,d,J=8.4 Hz).

### Industrial Applicability

The present invention can be used in the production of a 2-benzyl-5,5-bis(hydroxymethyl)-cyclopentanone derivative having a protected hydroxy group serving as a raw material for an agricultural chemical or the like.

## Claims

1. A production method for a cyclopentanone derivative represented by general formula (III) below: 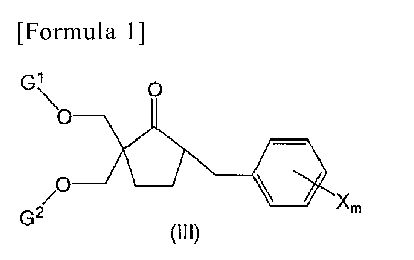 in formula (III), X is a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group; m is an integer from 0 to 5; a plurality of X moieties may be the same or different when m is 2 or greater; G¹ and G² each represents a protecting group that dissociates under acidic conditions, G¹ and G² may be the same or different, and G¹ and G² may bond with one another to form a ring;
the method comprising a step of obtaining a compound represented by general formula (II) below by reacting a compound represented by general formula (I) below with an acid: 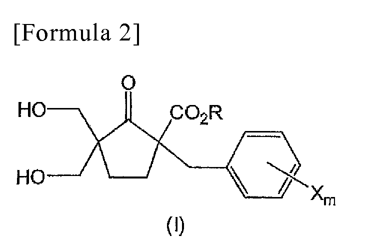 in formula (I), X and m are each the same as X and m in formula (III), and R is an alkyl group having from 1 to 4 carbons; and 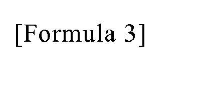 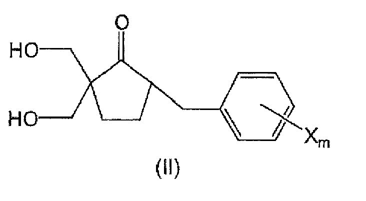 in formula (II), X and m are each the same as X and m in formula (III).

2. The production method for a cyclopentanone derivative according to claim 1, further comprising a step of obtaining the cyclopentanone derivative represented by general formula (III) above by protecting a hydroxy group of the compound represented by general formula (II) above with a protecting group that dissociates under acidic conditions.

3. The production method for a cyclopentanone derivative according to claim 1 or 2, wherein the cyclopentanone derivative represented by general formula (III) above is a compound represented by general formula (IIIa) below: 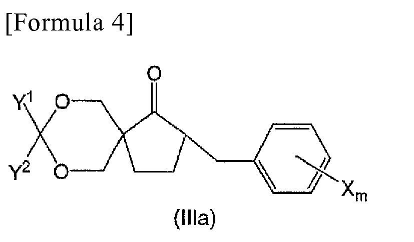 in formula (IIIa), X and m are each the same as X and m in formula (III); Y¹ and Y² are each independently a hydrogen atom, an alkyl group having from 1 to 4 carbons, an alkenyl group having from 1 to 4 carbons, or a substituted or unsubstituted phenyl group, naphthyl group, or benzyl group; and Y¹ and Y² may bond with one another to form a ring.

4. The production method for a cyclopentanone derivative according to claim 3, wherein Y¹ and Y² are each independently a hydrogen atom or an alkyl group having from 1 to 4 carbons.

5. The production method for a cyclopentanone derivative according to claim 3 or 4, wherein both Y¹ and Y² are methyl groups or hydrogen atoms.

6. The production method for a cyclopentanone derivative according to any one of claims 1 to 5, wherein in general formula (III) above, m is an integer from 0 to 3, and when m is 1 or greater, X is a halogen atom, an alkyl group having from 1 to 3 carbons, or a haloalkyl group having from 1 to 3 carbons.

7. The production method for a cyclopentanone derivative according to any one of claims 1 to 6, wherein in general formula (III) above, m is an integer from 0 to 2, and when m is 1 or 2, X is a halogen atom.

8. A production method for a compound represented by general formula (II) below: 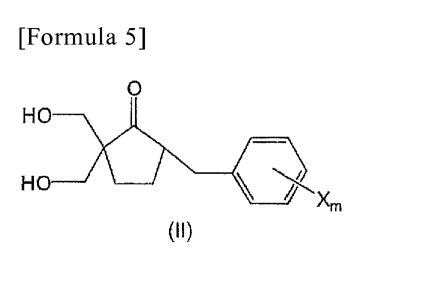 in formula (II), X is a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group; m is an integer from 0 to 5; and a plurality of X moieties may be the same or different when m is 2 or greater; and
wherein a compound represented by general formula (I) below is reacted with an acid: 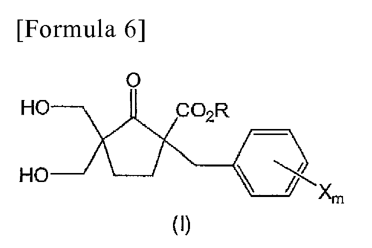 in formula (I), X and m are each the same as X and m in formula (II), and R is an alkyl group having from 1 to 4 carbons.

9. A production method for a cyclopentanone derivative represented by general formula (III) below: 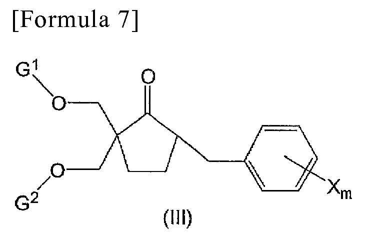 in formula (III), X is a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group; m is an integer from 0 to 5; a plurality of X moieties may be the same or different when m is 2 or greater; G¹ and G² each represents a protecting group that dissociates under acidic conditions, G¹ and G² may be the same or different, and G¹ and G² may bond with one another to form a ring;
a hydroxy group of a compound represented by general formula (II) below being protected by a protecting group that dissociates under acidic conditions: 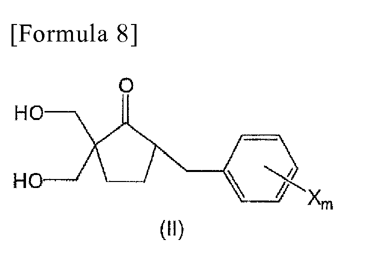 in formula (II), X and m are each the same as X and m in formula (III).

10. A compound represented by general formula (II) below: 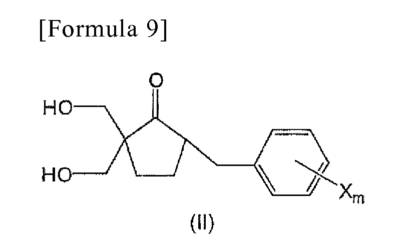 in formula (II), X is a halogen atom, an alkyl group having from 1 to 4 carbons, a haloalkyl group having from 1 to 4 carbons, an alkoxy group having from 1 to 4 carbons, a haloalkoxy group having from 1 to 4 carbons, a phenyl group, a cyano group, or a nitro group; m is an integer from 0 to 5; and a plurality of X moieties may be the same or different when m is 2 or greater.
